# EUROPEAN PATENT SPECIFICATION

(11) **EP 1 895 909 B1**
(45) Date of publication and mention of the grant of the patent: **10.08.2016**
(21) Application number: 06765766.8
(22) Date of filing: 15.06.2006
(51) Int. Cl.: A61B 8/14

(54) **METHOD AND APPARATUS FOR 3D ULTRASOUND IMAGING USING A STATIONARY BEAM TO ESTIMATE A PARAMETER**
VERFAHREN UND VORRICHTUNG ZUR DREIDIMENSIONALEN ULTRASCHALLBILDGEBUNG UNTER VERWENDUNG EINES FESTSTEHENDEN STRAHLS ZUM SCHÄTZEN EINES PARAMETERS
PROCEDE ET DISPOSITIF D'IMAGERIE ULTRASONIQUE 3D FAISANT INTERVENIR UN FAISCEAU STATIONNAIRE POUR ESTIMER UN PARAMETRE

(30) Priority: 23.06.2005 US 693469 P
(43) Date of publication of application: 12.03.2008
(73) Proprietor: Koninklijke Philips N.V., 5656 AE Eindhoven (NL)
(72) Inventor: SHERRILL, David S., Briarcliff Manor, New York 10510-8001 (US)
(74) Representative: de Haan, Poul Erik
(86) International application number: PCT/IB2006/051934
(87) International publication number: WO 2006/136988

(56) References cited:
- US-A1- 2005 049 503
- JING DENG ET AL: "Fetal echocardiography in three and four dimensions" Ultrasound in Medicine and Biology Elsevier USA, vol. 22, no. 8, 1996, pages 979-986, XP002405068 ISSN: 0301-5629
- DEVORE G R ET AL: "SPATIO-TEMPORAL IMAGE CORRELATION (STIC): NEW TECHNOLOGY FOR EVALUATION OF THE FETAL HEART" ULTRASOUND IN OBSTETRICS AND GYNECOLOGY, vol. 22, no. 4, 2003, pages 380-387, XP008031531
- SMITH S W ET AL: "Two-dimensional arrays for medical ultrasound", ULTRASONIC IMAGING, DYNAMEDIA INC., SILVER SPRING, MD, US, vol. 14, no. 3, 1 July 1992 (1992-07-01), pages 213-233, XP023045747, ISSN: 0161-7346, DOI: 10.1016/0161-7346(92)90064-3 [retrieved on 1992-07-01]

## Description

The present embodiments relate generally to medical ultrasound systems and more particularly, to a method and apparatus for 3D ultrasound imaging, for example, ultrasonic 3D fetal heart imaging.

In known methods for fetal heart imaging, an electrocardiogram (ECG) is not available. As a result, an ultrasound system uses spatial-temporal image correlation (STIC) to derive the cardiac phase from a spectral analysis of two-dimensional (2D) images while an imaging plane is being swept across an imaging volume. Using the cardiac phase derived from STIC, the ultrasound system rearranges the 2D images for three-dimensional (3D) processing. However, the accuracy of the STIC analysis varies from one imaging plane to another, especially if the heart rate does not remain steady.

With prior known methods similar to that described in Jing Deng et al: "Fetal Echocardiography in three and four dimensions", Ultrasound in Medicine and Biology, Elsevier USA, vol. 22, no. 8, 1996, pp. 979-986, XP002405068 ISSN: 0301-5629, Fetal STIC imaging includes constructing 3D views of the fetal heart from images acquired over many heart beats. In addition, the current data processing techniques for Fetal STIC imaging require that the heart rate remain steady. However, odd beats or heart rate changes can degrade the 3D views by causing information from different cardiac phases to be intermingled in views that should represent single cardiac phases.

Accordingly, an improved method and ultrasound system for overcoming the problems in the art is desired.

According to claim 1, the present disclosure provides a method of three-dimensional (3D) ultrasound imaging comprising:
using a transducer with 3D electronic steering to acquire ultrasound data representative of an imaging volume as a function of time, from which can be obtained a plurality of two-dimensional images, the transducer being configured (i) for electronically steering ultrasound beams to acquire data representative of a 2D ultrasound image within a 2D imaging plane of the imaging volume and (ii) for sweeping the 2D imaging plane across the imaging volume;
acquiring data from a stationary ultrasound beam concurrently with the acquiring of the ultrasound data representative of the imaging volume;
analyzing the stationary ultrasound beam data to derive a parameter from the stationary ultrasound beam data; and
rearranging a plurality of 2D ultrasound images obtained from the acquired ultrasound data representative of the imaging volume as a function of the derived parameter.

The step of acquiring data from the stationary ultrasound beam comprises using said transducer with 3D electronic steering, wherein the transducer with 3D electronic steering is further configured for (iii) interleaving the acquiring data from the stationary ultrasound beam with the acquiring of the 2D ultrasound images, and wherein the stationary ultrasound beam concurrently acquired with the ultrasound data representative of the imaging volume has the same stationary location through the imaging volume for all two dimensional (2D) ultrasound images.

Further, a corresponding three-dimensional (3D) ultrasound imaging apparatus is provided that comprises:
a control unit; and
an ultrasound transducer coupled to the control unit, wherein said control unit is configured for (i) controlling the ultrasound transducer and (ii) performing 3D ultrasound imaging according to the above-mentioned method.

In one embodiment, acquiring data from the stationary ultrasound beam comprises one or more of an M-mode acquisition, a Doppler mode acquisition, or an acquisition tailored to a specific ultrasound imaging application. The method can also be implemented by an ultrasound imaging system, as well as in the form of a computer program product.
Figure 1 is a partial block diagram view of an ultrasound system according to an embodiment of the present disclosure;
Figure 2 is a simplified schematic diagram view illustrating 3D ultrasound imaging of a target volume with use of the ultrasound imaging system and method according to an embodiment of the present disclosure; and
Figure 3 is allow diagram view illustrating a method of 3D ultrasound imaging according to another embodiment of the present disclosure.

In the figures, like reference numerals refer to like elements. In addition, it is to be noted that the figures may not be drawn to scale.

As discussed herein above, prior known methods of Fetal STIC imaging have involved constructing 3D views of the fetal heart from images acquired over many heart beats. In addition, the data processing techniques for the prior Fetal STIC imaging methods required that the heart rate remain steady, however, odd beats or heart rate changes degrade the 3D views by causing information from different cardiac phases to be intermingled in views that should represent single cardiac phases. In contrast, according to one embodiment of 3D ultrasound imaging of the present disclosure, the method includes (i) monitoring the heart to determine the actual cardiac phase of each 2D image and (ii) using the determined cardiac phase information to avoid mixing different phases within a single 3D view. Monitoring of the heart is accomplished by using ultrasound, and more particularly, using a stationary ultrasound beam and wherein the transducer remains stationary. Accordingly, Doppler mode or M-mode acquisition can be used to monitor a chosen anatomical location on a fine time scale.

It is further noted that use of Doppler mode or M-mode acquisition is not possible with prior Fetal STIC imaging methods which use mechanical transducer motion to acquire the 3D volume for Fetal STIC. In other words, moving the transducer makes it impossible to hold a Doppler or M-mode line on the chosen anatomical location. The methods according to the embodiments of the present disclosure include a realization that new transducers that scan 3D volumes without mechanical motion (for example, 2D array or matrix transducers) allow for overcoming this limitation, That is a transducer that scans 3D volumes without mechanical motion can be configured to transmit lines throughout a 3D volume. In addition, the transducer capable of 3D scanning without mechanical movement, hereinafter referred to as a transducer with 3D electronic steering, can further be configured to interleave a stationary monitor pulse with pulses used to acquire a 3D volume, for example, in connection with fetal STIC.

In order to scan a 3D volume using a transducer that cannot electronically steer throughout the volume, hereinafter referred to as a transducer without 3D electronic steering, the transducer must be mechanically moved. For example, a 3D volume can be scanned using a 1D phased array transducer by moving the transducer so that its 2D scan plane moves across the volume being scanned. As indicated herein above, moving the transducer without 3D electronic steering in this way makes it impossible to maintain a stationary monitor beam. The situation is much the same with so-called 1.5D transducers, which have some ability to control elevation focusing or elevation steering or both. In contrast, a transducer with 3D electronic steering remains stationary and uses electronic steering to sweep through a 3D volume, whereas a transducer without 3D electronic steering must move to sweep through the 3D volume.

A review of 2D ultrasound imaging, M-mode, and Doppler data acquisition is provided below. It is noted that the numbers included herein represent but a single example of how this may be done, and other numbers may be used. For simple 2D ultrasound imaging, image data can be acquired from 50 transmit lines spanning a 90 degree wedge. The frame rate can be 50 Hz, or 20 ms per frame. Time between adjacent lines is 0.4 ms. In addition, time between repeated views of the same line is the frame time, 20 ms.

With respect to M-mode acquisition, M-mode is used to see motion on finer time scales. For example, a system operator selects one line from a 2D frame. The seleeted line is thereafter acquired 5 times evenly spaced (in time) among the 50 lines of the 2D frame. The scanner will acquire ten 2D lines and then re-acquire the M-mode line, so that the M-mode view is updated every 4 ms. Accordingly, the M-mode acquisition enables seeing motion on smaller time scales than with 2D imaging alone, in which the image updates only once every 20 ms. An M-mode trace is composed by displaying the acquired M-mode lines side-by-side. The M-mode trace includes a scrolling trace, similar to a strip-chart recorder. In addition, with respect to the M-mode trace, the vertical axis represents depth and the horizontal axis represents time.

Duplex Doppler uses a similar acquisition strategy to that of M-mode acquisition; however, a duplex Doppler line would typically be acquired after every 2D image line. In addition, for Doppler acquisition, the acquired data is used to determine blood flow rather than being used to form a spatial image. The vertical axis of a Doppler acquisition trace represents velocity (of the moving blood) and the horizontal axis represents time. In addition, it is noted that a cardiac cycle is evident in both M-mode and Doppler traces.

Furthermore, while using duplex Doppler drops the 2D frame rate by about half, it would almost certainly be unacceptable for use with known fetal STIC imaging methods. However, with the method of 3D imaging according to the embodiments of the present disclosure, duplex Doppler provides for a fetal STIC improvement, by employing acquisition timing similar to M-mode.

According to one embodiment of the present disclosure, a method of 3D ultrasound imaging includes using a transducer with 3D electronic steering (for example, a 2D array transducer) without additional steering via mechanical movement, wherein derivation of cardiac phase is improved by analyzing a stationary ultrasound beam instead of a collection of imaging planes (comprising a 3D volume). The same stationary ultrasound beam can be used for all image planes, for example, during a particular fetal heart imaging procedure, so that consistent results are obtained for all imaging planes. In other words, the embodiments of the present disclosure correctly determine the cardiac phase of each image even if the heart rate changes during the cardiac acquisition. According to one embodiment, the stationary beam can include an M-mode acquisition, a Doppler acquisition, or an acquisition tailored specifically to fit the ultrasound imaging requirements of a particular imaging procedure. The monitor beam may be acquired less often than is typical for either M-mode or Doppler, perhaps only once per 2D image frame, and perhaps even less often.

According to one embodiment of the present disclosure, an ultrasound system comprises a transducer with 3D electronic steering configured to interleave a sweep of 2D data acquisitions with a stationary acquisition, wherein the stationary acquisition comprises an M-mode acquisition or a Doppler acquisition. As noted herein, transducers that are mechanically swept to perform 3D acquisitions, either by motorization or by other manipulation, are not able to interleave a sweep of 2D data acquisitions with a stationary acquisition.

The embodiments of the present disclosure can be implemented by deriving the cardiac phase from a SIC analysis of the M-mode and/or Doppler data streams obtained with the use of the stationary ultrasound beam. Alternatively, the STIC algorithms may be modified for better performance with M-mode and/or Doppler data streams. Furthermore an additional new approach to the analysis can be implemented for better performance with M-mode and/or Doppler data streams. Moreover, a novel form of acquisition tailored specifically to fit the 3D ultrasound imaging application may be used either in place of or in addition to M-mode and/or Doppler data streams. Furthermore, as discussed herein, the embodiments of the present disclosure can be implemented in ultrasound systems that support both 3D fetal heart imaging and matrix (2D array) ultrasound transducers.

According to another embodiment, a method of three-dimensional (3D) ultrasound imaging comprises acquiring ultrasound data representative of an imagine volume as a function of time, from which can be obtained a plurality of two dimensional (2D) ultrasound images. The method further comprises acquiring data from a stationary ultrasound beam concurrently with the acquiring of the ultrasound data representative or the imaging volume. The stationary ultrasound beam data is analysed to derive a parameter from the stationary ultrasound beam data. In addition, the acquired ultrasound data is rearranged for 3D processing as a function of the derived parameter. From the rearranged ultrasound data one or more 2D images ordered according to the derived parameter may be obtained. Likewise, from the rearranged ultrasound data one or more 3D surface rendered images ordered according to the derived parameter may be obtained. Acquiring of the ultrasound data can further comprise one or more of (i) a consecutive acquisition order across the imaging volume, (ii) a non-consecutive acquisition order across the imaging volume, or (iii) a prescribed acquisition order across the imaging volume. The prescribed acquisition order can include any arbitrary order selected according to the requirements of a particular acquisition.

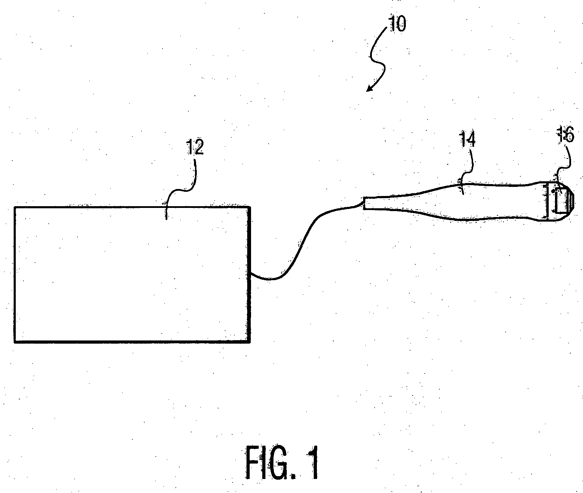

Referring now to the drawings, Figure 1 is a block diagram view of a three-dimensional (3D) ultrasound imaging system 10 according to an embodiment of the present disclosure. The 3D ultrasound imaging system 10 includes a control or base unit 12 configured for use with an ultrasound transducer probe 14, further for carrying out the ultrasound imaging methods as discussed herein according to the embodiments of the present disclosure. The probe 14 contains an ultrasound transducer 16. In one embodiment, the control unit 12 is configured for (i) controlling the ultrasound transducer 16 and (ii) performing 3D ultrasound imaging according to the 3D ultrasound imaging methods of the present disclosure.

In one embodiment, ultrasound transducer 16 comprises a matrix transducer, also referred to as a two-dimensional array transducer. Furthermore, base unit 12 includes suitable control electronics for performing 3D ultrasound imaging as discussed herein. For example, in one embodiment, base unit 12 can comprises a computer as discussed further herein. Ultrasound transducer probe 14 couples to base unit 12 via a suitable connection, for example, an electronic cable, a wireless connection, or other suitable means.

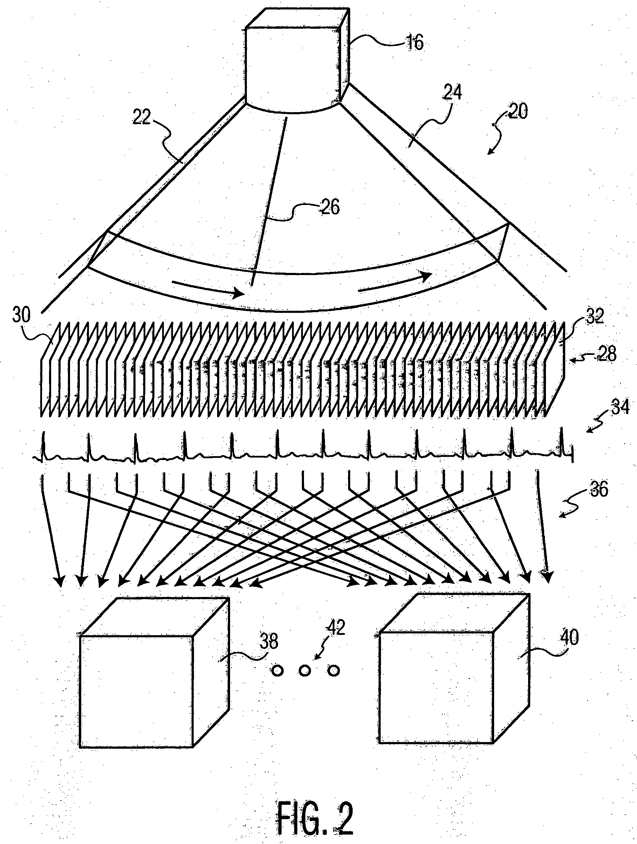

Figure 2 is a simplified schematic diagram view illustrating 3D ultrasound imaging of a target volume with use of the ultrasound imaging system 10 according to an embodiment of the present disclosure. In particular, ultrasound transducer 16 produces a sweep 20 of ultrasound beams of a 2D imaging plane directed into an imaging volume (not shown) in response to an activation signal from base unit 12. For example, the sweep 20 can comprise a sweep from an initial 2D imaging plane 22 to a final 2D imaging plane 24. The ultrasound energy can be adjusted as needed, for example by a repositioning of the ultrasound transducer 16 (via repositioning of probe 14) with respect to the target location or imaging volume and/or through appropriate activation signals from base unit 12, according to the requirements of a particular 3D ultrasound imaging application. In addition, the imaging volume is disposed in a region of interest within a subject to be imaged according to the methods of the present disclosure.

According to one embodiment of the present disclosure, the method of three-dimensional (3D) ultrasound imaging comprises acquiring a plurality of two-dimensional (2D) ultrasound images 28 as a 2D imaging plane is swept across an imaging volume. For example, the 2D ultrasound images 28 include images from an initial image 30 to a final image 32, corresponding to the sweep 20 from the initial 3D imaging plane 22 to the final imaging plane 24. Concurrently with the acquiring of the plurality of 2D ultrasound images, data from a stationary ultrasound beam 26 is acquired. The stationary ultrasound beam data is analyzed to derive a parameter 34 from the stationary ultrasound beam data. Furthermore, the 2D ultrasound images are rearranged into new groups of images, as indicated by reference numeral 36 in Figure 2, for 3D processing as a Junction of the derived parameter. Within the new groups there are a number of images. As shown in Figure 2, for illustration only, the new groups include eleven 20 images. The base unit 12 of 3D ultrasound system is configured for arranging the images spatially within the new groups because the images have all occurred at different positions in space with known positional co-ordinates. In the example of Figure 2, two volumes 38 any 49 are shown. One volume 3 8 at systole and another volume 40 at diastole. In addition, there can exist additional volumes between these two volumes (38, 40) and their corresponding locations, as illustrated by reference numeral 42.

In one embodiment, the derived parameter comprises a cardiac phase. In other words, the imaging volume contains a cardiac source, the cardiac source having a number of cardiac phases. For example, the cardiac source can comprise a fetal heart.

According to another embodiment, acquiring the plurality of 2D ultrasound images comprises using a matrix transducer. The matrix transducer can be configured (i) for electronically steering ultrasound beams to acquire 2D ultrasound images and (ii) for sweeping the 2D imaging plane across the imaging volume. In addition, acquiring the stationary ultrasound beam data can also comprise using the matrix transducer, wherein the matrix transducer is further configured for (iii) interleaving the acquiring of the 2D ultrasound images with stationary ultrasound beam data acquisition.

In one embodiment, the transducer with 3D electronic steering 16 is configured for steering the stationary ultrasound beam 26 to occur within the imaging volume at a position for obtaining an optimal signal. That is, the stationary ultrasound beam 26 can be steered, and a positioning of the stationary ultrasound beamed be adjusted as may be necessary, to an optimal or other suitable location within the imaging volume to improve derivation of the parameter for the acquiring of the ultrasound data representative of the imaging volume and the obtaining of the plurality of 2D ultrasound images 28. For example, a positioning of the stationary ultrasound beam may be adjusted during an imaging volume acquisition sequence to provide a desired tracking of a cardiac phase. Furthermore, acquiring of the stationary ultrasound beam data can comprise, for example, an M-mode acquisition or a Doppler mode acquisition. In another embodiment, acquiring the stationary ultrasound beam data can comprise an acquisition tailored to a specific ultrasound imaging application.

In another embodiment, acquiring the stationary ultrasound beam data comprises acquiring the stationary ultrasound beam data concurrently with the acquiring of each of the plurality of 2D ultrasound images 28. Accordingly, analyzing the stationary ultrasound beam data includes analyzing data from each respective stationary ultrasound beam data acquisition to derive the parameter 34 for a corresponding 2D ultrasound image. In one embodiment, analyzing further includes performing a spatial-temporal image correlation (STIC) analysis of the stationary ultrasound beam data, with appropriate adaptation of the STIC methods to the acquired data. The stationary ultrasound beam data can comprise, for example, one or more of an M-mode data stream, a Doppler mode data stream, or other data stream. In addition, a same stationary ultrasound beam 26 can be used concurrently for all 2D imaging planes to enable a consistent derivation of the parameter for the plurality of 2D ultrasound images.

In another embodiment, a method of three-dimensional (3D) ultrasound imaging comprises acquiring a plurality of two-dimensional (2D) ultrasound images as a 2D imaging plane is swept across an imaging volume that contains a cardiac source, the cardiac source having a number of cardiac phases, and wherein acquiring the plurality of 2D ultrasound images comprises using a transducer with 3D electronic steering configured (i) for electronically steering ultrasound beams to acquire 2D ultrasound images and (ii) for sweeping the 2D imaging plane across the imaging volume. The method further comprises acquiring data from a stationary ultrasound beam concurrently with the acquiring of the plurality of 2D ultrasound images, wherein acquiring the stationary ultrasound beam data further comprises using the matrix transducer, wherein the transducer with 3D electronic steering is further configured for (iii) interleaving the acquiring of the 2D ultrasound images with stationary ultrasound beam data acquisition. In addition, the stationary ultrasound beam data is analyzed to derive a cardiac phase from the stationary ultrasound beam data. Furthermore, the 2D ultrasound images are rearranged for 3D processing as a function of the derived cardiac phase.

In the embodiment of the preceding paragraph, the acquiring of the stationary ultrasound beam data can comprise one or more of an M-mode acquisition, a Doppler mode acquisition, or an acquisition tailored to a specific ultrasound imaging application. In addition, in another embodiment, the analyzing includes performing a spatial-temporal image correlation (STIC) analysis of the stationary ultrasound beam data. Still further, in another embodiment a same stationary ultrasound beam is used concurrently for all 2D imaging planes to enable a consistent derivation of the cardiac phase for the plurality of 2D ultrasound images. Furthermore, the stationary ultrasound beam is selectively positioned for obtaining an optimal signal to improve derivation of the cardiac phase for the plurality of 2D ultrasound images.

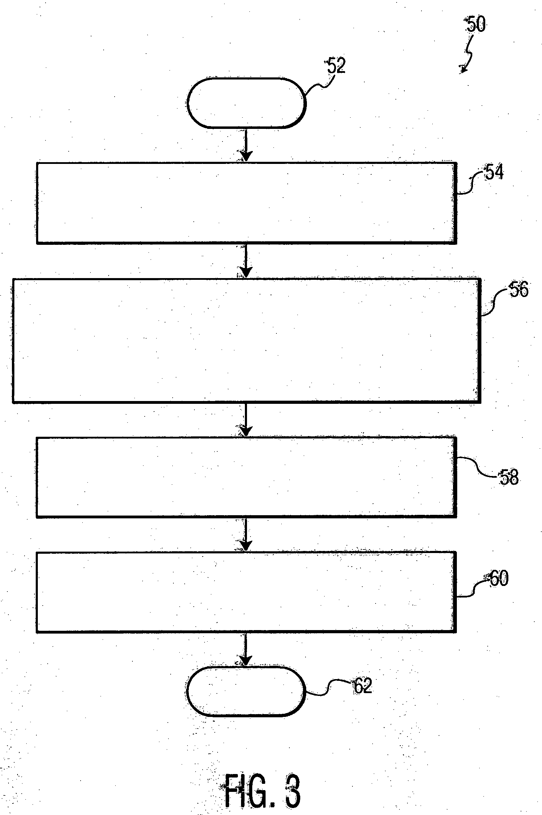

Figure 3 is a flow diagram view illustrating a method of 3D ultrasound imaging, generally indicated by reference numeral 50, according to another embodiment of the present disclosure. The method begins with step 52, wherein initial actions are taken by a system operator in setting up the ultrasound imaging equipment in preparation for acquiring a 3D ultrasound image of a desired imaging volume. At step 54, the method includes acquiring a plurality of two-dimensional (2D) ultrasound images as a 2D imaging plane is swept across an imaging volume. At step 56, concurrently with the acquiring of the plurality of 2D ultrasound images, the method includes acquiring data from a stationary ultrasound beam. At step 5 8, the stationary ultrasound beam data is analyzed to derive a parameter from the stationary ultrasound beam data. In one embodiment, the parameter includes a cardiac phase. At step 60, the method includes rearranging the 2D ultrasound images for 3D processing as a function of the derived parameter. Additional processing, as may be appropriate for a particular 3D ultrasound imaging application, continues and/or occurs with steep 62.

In addition to the above, the embodiments of the present disclosure also include computer software or a computer program product. The computer program product includes a computer readable media having a set of instructions executable by a computer for carrying out the methods of 3D ultrasound imaging as described and discussed herein. The computer readable media can include any suitable computer readable media for a given ultrasound imaging system application. Still further, the computer readable media may include a network communication media. Examples of network communication media include, for example, an intranet, the Internet, or an extranet. In one embodiment, control unit 12 can comprise a computer.

Although only a few exemplary embodiments have been described in detail above, those skilled in the art will readily appreciate that many modifications are possible in the exemplary embodiments without materially departing from the novel teachings and advantages of the embodiments of the present disclosure. For example, the embodiments of the present disclosure can be applied to 3D ultrasound imaging such as 3D fetal heart ultrasound imaging. Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements, and/or by means of a suitably programmed computer. In a device claim enumerating several means, several of these means may be embodied by one and the same item of hardware. The mere fact that certain measures are recited in mutually different dependent claims does not indicate that a combination of these measures cannot be used to an advantage.

## Claims

1. A method of three-dimensional (3D) ultrasound imaging comprising:
using a transducer (16) with 3D electronic steering to acquire ultrasound data representative of an imaging volume as a function of time (20), from which can be obtained a plurality of two dimensional (2D) ultrasound images (28), the transducer (16) being configured (i) for electronically steering ultrasound beams to acquire data representative of a 2D ultrasound image within a 2D imaging plane of the imaging volume and (ii) for sweeping the 2D imaging plane across the imaging volume;
acquiring data from a stationary ultrasound beam concurrently with the acquiring of the ultrasound data representative of the imaging volume;
analyzing the stationary ultrasound beam data to derive a parameter (34) from the stationary ultrasound beam data; and
rearranging a plurality of two dimensional (2D) ultrasound images obtained from the acquired ultrasound data representative of the imaging volume as a function of the derived parameter,
**characterized in that**
acquiring data from the stationary ultrasound beam (26) comprises using said transducer (16) with 3D electronic steering, wherein the transducer with 3D electronic steering is further configured for (iii) interleaving the acquiring data from the stationary ultrasound beam with the acquiring of the 2D ultrasound images, and **in that** the stationary ultrasound beam concurrently acquired with the ultrasound data representative of the imaging volume has the same stationary location through the imaging volume for all two dimensional (2D) ultrasound images.

2. The method of claim 1, wherein acquiring the stationary ultrasound beam data comprises one or more of an M-mode acquisition, a Doppler mode acquisition, and an acquisition tailored to a specific ultrasound imaging application.

3. The method of claim 1, wherein acquiring the stationary ultrasound beam data comprises acquiring the stationary ultrasound beam data concurrently with the acquiring of ultrasound data for each of the plurality of 2D ultrasound images, and wherein analyzing the stationary ultrasound beam data comprises analyzing data from each respective stationary ultrasound beam data acquisition to derive the parameter for a corresponding 2D ultrasound image.

4. The method of claim 3, wherein analyzing further includes performing a spatial-temporal image correlation (STIC) analysis of the stationary ultrasound beam data.

5. The method of claim 1, further comprising:
adjusting a positioning of the stationary ultrasound beam for obtaining an optimal signal to improve derivation of the parameter for the plurality of 2D ultrasound images.

6. The method of claim 1, wherein acquiring the ultrasound data comprises one or more of (i) a consecutive acquisition order across the imaging volume, and (ii) a non-consecutive acquisition order across the imaging volume, and (iii) a prescribed acquisition order across the imaging volume.

7. A three-dimensional (3D) ultrasound imaging apparatus comprising:
a control unit (12); and
an ultrasound transducer (16) coupled to the control unit, wherein said control unit is configured for (i) controlling the ultrasound transducer and (ii) performing 3D ultrasound imaging according to the method of claim 1.

8. The method of claim 1, wherein
the imaging volume contains a cardiac source, the cardiac source having a number of cardiac phases, and wherein
the stationary ultrasound beam data is analyzed to derive a cardiac phase; and
the plurality of two dimensional (2D) ultrasound images is rearranged as a function of the derived cardiac phase.

## Patentansprüche

1. Verfahren zur dreidimensionalen (3D) Ultraschallbildgebung, das Folgendes umfasst:
Verwenden eines Wandlers (16) mit elektronischer dreidimensionaler Steuerung zum Erfassen von für ein Bildgebungsvolumen repräsentativen Ultraschalldaten als eine Funktion der Zeit (20), aus denen eine Vielzahl von zweidimensionalen (2D) Ultraschallbildern (28) gewonnen werden kann, wobei der Wandler (16) konfiguriert ist, um (i) die Ultraschallstrahlen elektronisch zu steuern, damit für ein zweidimensionales Ultraschallbild innerhalb einer zweidimensionalen Bildgebungsebene des Bildgebungsvolumen repräsentative Daten erfasst werden, und um (ii) die zweidimensionale Bildgebungsebene über das Bildgebungsvolumen hinweg abzutasten;
Erfassen von Daten von einem feststehenden Ultraschallstrahl gleichzeitig mit dem Erfassen von Ultraschalldaten, die für das Bildgebungsvolumen repräsentativ sind;
Analysieren der feststehenden Ultraschallstrahlendaten, um einen Parameter (34) von den feststehenden Ultraschallstrahlendaten abzuleiten; und
neues Anordnen einer Vielzahl von zweidimensionalen (2D) Ultraschallbildern, die aus den erfassten für das Bildgebungsvolumen repräsentativen Ultraschalldaten gewonnen wurden, als Funktion des abgeleiteten Parameters,
**dadurch gekennzeichnet, dass**
das Erfassen von Daten von dem feststehenden Ultraschallstrahl (26) das Verwenden des genannten Wandlers (16) mit elektronischer dreidimensionaler Steuerung umfasst, wobei der Wandler mit elektronischer dreidimensionaler Steuerung weiterhin konfiguriert ist, um (iii) die erfassten Daten von dem feststehenden Ultraschallstrahl mit der Erfassung der zweidimensionalen Ultraschalldaten zu verschachteln, und dadurch, dass der feststehende Ultraschallstrahl, der gleichzeitig mit den Ultraschalldaten erfasst wird, die für das Bildgebungsvolumen repräsentativ sind, über das Bildgebungsvolumen hinweg den gleichen feststehenden Ort für alle zweidimensionalen (2D) Ultraschallbilder hat.

2. Verfahren nach Anspruch 1, wobei das Erfassen der feststehenden Ultraschallstrahlendaten eine oder mehrere von folgenden Erfassungsarten umfasst: M-Mode-Erfassung, Doppler-Mode-Erfassung und eine Erfassung, die auf eine spezifische Ultraschallbildgebungsanwendung zugeschnitten ist.

3. Verfahren nach Anspruch 1, wobei das Erfassen der feststehenden Ultraschallstrahlendaten das Erfassen der feststehenden Ultraschallstrahlendaten gleichzeitig mit dem Erfassen von Ultraschalldaten für jedes der Vielzahl von zweidimensionalen Ultraschallbildern umfasst, und wobei das Analysieren der feststehenden Ultraschallstrahlendaten das Analysieren der Daten von jeder betreffenden feststehenden Ultraschallstrahlendatenerfassung zum Ableiten des Parameters für ein entsprechendes zweidimensionalen Ultraschallbild umfasst.

4. Verfahren nach Anspruch 3, wobei das Analysieren weiterhin das Durchführen einer räumlich-zeitlichen Bildkorrelationsanalyse (engl. spatial-temporal image correlation, STIC) der feststehenden Ultraschallstrahlendaten umfasst.

5. Verfahren nach Anspruch 1, das weiterhin Folgendes umfasst:
Einstellen einer Positionierung des feststehenden Ultraschallstrahls zum Erlangen eines optimalen Signals zum Verbessern der Ableitung des Parameters für die Vielzahl von zweidimensionalen Ultraschallbildern.

6. Verfahren nach Anspruch 1, wobei das Erfassen von Ultraschalldaten eine oder mehrere von folgenden Erfassungsreihenfolgen umfasst: (1) eine konsekutive Erfassungsreihenfolge über das Bildgebungsvolumen, und (ii) eine nicht-konsekutive Erfassungsreihenfolge über das Bildgebungsvolumen, und (iii) eine vorgeschriebene Erfassungsreihenfolge über das Bildgebungsvolumen.

7. Gerät zur dreidimensionalen (3D) Ultraschallbildgebung, das Folgendes umfasst:
eine Steuereinheit (12); und
einen Ultraschallwandler (16), der mit der Steuereinheit gekoppelt ist, wobei die genannte Steuereinheit konfiguriert ist, um (i) den Ultraschallwandler zu steuern und (ii) die dreidimensionale Ultraschallbildgebung nach dem Verfahren aus Anspruch 1 durchzuführen.

8. Verfahren nach Anspruch 1, wobei
das Bildgebungsvolumen eine kardiale Quelle enthält, wobei die kardiale Quelle eine Anzahl von kardialen Phasen hat, und wobei
die feststehenden Ultraschallstrahlendaten analysiert werden, um eine kardiale Phase abzuleiten; und
die Vielzahl von zweidimensionalen (D) Ultraschallbildern als Funktion der abgeleiteten kardialen Phase neu angeordnet wird.

## Revendications

1. Procédé d'imagerie ultrasonique tridimensionnelle (3D) comprenant :
l'utilisation d'un transducteur (16) avec une commande électronique 3D pour acquérir des données ultrasoniques représentatives d'un volume d'imagerie en fonction du temps (20), à partir desquelles on peut obtenir une pluralité d'images ultrasoniques bidimensionnelles (2D) (28), le transducteur (16) étant configuré (i) pour commander électroniquement des faisceaux ultrasoniques pour acquérir des données représentatives d'une image ultrasonique 2D dans un plan d'imagerie 2D du volume d'imagerie et (ii) pour balayer le plan d'imagerie 2D à travers le volume d'imagerie ;
l'acquisition de données à partir d'un faisceau ultrasonique fixe en même temps que l'acquisition des données ultrasoniques représentatives du volume d'imagerie ;
l'analyse des données de faisceau ultrasonique fixe pour dériver un paramètre (34) des données de faisceau ultrasonique fixe ; et
le réagencement d'une pluralité d'images ultrasoniques bidimensionnelles (2D) obtenues à partir des données ultrasoniques acquises représentatives du volume d'imagerie en fonction du paramètre dérivé,
**caractérisé en ce que**
l'acquisition de données à partir du faisceau ultrasonique fixe (26) comprend l'utilisation dudit transducteur (16) avec une commande électronique 3D, dans lequel le transducteur avec une commande électronique 3D est en outre configuré pour (iii) intercaler l'acquisition de données issues du faisceau ultrasonique fixe avec l'acquisition des images ultrasoniques 2D, et **en ce que** le faisceau ultrasonique fixe acquis en même temps que les données ultrasoniques représentatives du volume d'imagerie a le même emplacement fixe à travers le volume d'imagerie pour toutes les images ultrasoniques bidimensionnelles (2D).

2. Procédé selon la revendication 1, dans lequel l'acquisition des données de faisceau ultrasonique fixe comprend une ou plusieurs parmi une acquisition en mode M, une acquisition en mode Doppler et une acquisition adaptée à une application d'imagerie ultrasonique spécifique.

3. Procédé selon la revendication 1, dans lequel l'acquisition des données de faisceau ultrasonique fixe comprend l'acquisition des données de faisceau ultrasonique fixe en même temps que l'acquisition de données ultrasoniques pour chaque image de la pluralité d'images ultrasoniques 2D, et dans lequel l'analyse des données de faisceau ultrasonique fixe comprend l'analyse de données provenant de chaque acquisition de données de faisceau ultrasonique fixe respective pour dériver le paramètre pour une image ultrasonique 2D correspondante.

4. Procédé selon la revendication 3, dans lequel l'analyse inclut en outre la réalisation d'une analyse par corrélation d'image spatio-temporelle (STIC) des données de faisceau ultrasonique fixe.

5. Procédé selon la revendication 1, comprenant en outre :
l'ajustement d'un positionnement du faisceau ultrasonique fixe pour obtenir un signal optimal afin d'améliorer la dérivation du paramètre pour la pluralité d'images ultrasoniques 2D.

6. Procédé selon la revendication 1, dans lequel l'acquisition des données ultrasoniques comprend un ou plusieurs parmi (i) un ordre d'acquisition consécutif à travers le volume d'imagerie, et (ii) un ordre d'acquisition non consécutif à travers le volume d'imagerie et (iii) un ordre d'acquisition prescrit à travers le volume d'imagerie.

7. Appareil d'imagerie ultrasonique tridimensionnelle (3D) comprenant :
une unité de commande (12) ; et
un transducteur ultrasonique (16) couplé à l'unité de commande, dans lequel ladite unité de commande est configurée pour (i) commander le transducteur ultrasonique et (ii) réaliser l'imagerie ultrasonique 3D selon le procédé de la revendication 1.

8. Procédé selon la revendication 1, dans lequel
le volume d'imagerie contient une source cardiaque, la source cardiaque ayant un certain nombre de phases cardiaques, et dans lequel
les données de faisceau ultrasonique fixe sont analysées pour dériver une phase cardiaque ; et
la pluralité d'images ultrasoniques bidimensionnelles (2D) sont réagencées en fonction de la phase cardiaque dérivée.
